(19) **Europäisches Patentamt European Patent Office Office européen des brevets**

(11) **EP 4 185 345 B1**

(12) **EUROPEAN PATENT SPECIFICATION**

(45) Date of publication and mention
of the grant of the patent:
**17.12.2025 Bulletin 2025/51**

(21) Application number: **21847010.2**

(22) Date of filing: **23.07.2021**

(51) International Patent Classification (IPC):
**A61M 1/00** *(2006.01)*   **B09B 3/00** *(2022.01)*
**B65D 81/32** *(2006.01)*   **B65D 51/28** *(2006.01)*

(52) Cooperative Patent Classification (CPC):
**A61M 1/60; A61M 1/882;** A61J 1/1412;
A61J 1/2027

(86) International application number:
**PCT/US2021/043041**

(87) International publication number:
**WO 2022/020761 (27.01.2022 Gazette 2022/04)**

(54) **COVERING ASSEMBLY WITH COAGULANT COMPARTMENT AND USES THEREOF IN A BLOOD MONITORING/MANAGEMENT SYSTEM**

**ABDECKUNGSANORDNUNG MIT KOAGULATIONSKAMMER UND VERWENDUNGEN DAVON IN EINEM BLUTÜBERWACHUNGS-/-MANAGEMENTSYSTEM**

**ENSEMBLE COUVERCLE AVEC COMPARTIMENT DE COAGULANT ET SES UTILISATIONS DANS UN SYSTÈME DE SURVEILLANCE/GESTION DU SANG**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **23.07.2020 US 202063055577 P**
**18.09.2020 US 202017026106**

(43) Date of publication of application:
**31.05.2023 Bulletin 2023/22**

(60) Divisional application:
**23158031.7 / 4 215 225**

(73) Proprietor: **Cypher Medical, LLC**
**Bandera, TX 78003 (US)**

(72) Inventor: **CAREW, Christopher A.**
**Fair Oaks Ranch, TX 78015 (US)**

(74) Representative: **Bradbury, Simon Timothy Nicholas**
**Withers & Rogers LLP**
**2 London Bridge**
**London SE1 9RA (GB)**

(56) References cited:
WO-A1-2021/054297    CN-Y- 201 070 460
JP-A- H07 167 716    US-A- 5 185 007
US-A1- 2011 163 091    US-A1- 2011 163 091
US-A1- 2018 008 757    US-A1- 2018 196 031
US-A1- 2019 302 096

**Description**

CROSS-REFERENCE TO RELATED APPLICATIONS

This application claims the benefit of United States application no. 17/026,106, filed 18 September 2020, which claims priority to United States provisional application no. 63/055,577, filed 23 June 2020.

FIELD OF THE INVENTION

The present invention is related to the field of lids and/or coverings for a container.

BACKGROUND OF THE INVENTION

The containment and management of waste and other materials with potentially biohazardous materials, such as blood, requires careful handling. The presence of blood in fluids and other materials, such as materials generated during processing and/or procedures with animal and/or human bodies and/or corpses (e.g., surgery, taxidermy, corpse handling, processing and/or management (mortuary, morgue, hospital, veterinary office, taxidermy, tissue banks), requires that procedures and equipment be utilized to minimize contact and/or spillage generally of the fluid and/or waste. The problem of fluid/waste spilling and contact creates significant risk of exposing a patient, attending clinical personnel, and a surrounding surgical environment to blood contaminants. Existing collection vessels/containers used in materials collection and disposal do not effectively control for liquid/waste contaminant exposure, due at least in part to the fluid state in which any blood present in the collected material exists.

Containers for collecting biologically hazardous materials, particularly those materials collected from surgical procedures, may include a lid that includes a port that must be manually opened by a user, and manually emptied by the user, in order to dispose of the liquid waste contents. This procedure creates risk of contaminating the user performing the disposal activity, risk of contaminating others within the immediate area where the contents are being disposed of, and risk of contaminating the area in general where the procedure is being performed. Safer disposal methods for handling and discarding liquid or semi-liquid waste materials are needed, especially in the medical arts, to guard against these potential and significant health safety risks and/or other hazards.

The liquidity of a collected material is associated, at least in part, with the liquid state in which any blood in the material remains for several hours under standard room temperature and collection conditions. Any blood present may remain in a liquid state for much longer time periods, depending on what other materials are present. Techniques for quickly and easily reducing the liquidity of collected material, particularly biohazardous materials, remain largely underdeveloped to meet current needs.

Blister packaging sub-assemblies or "blister packs", have been used for convenient packaging of a variety of consumer products, such as chewing gum, throat lozenges, certain medications, and the like. As used in this sense, a "blister pack" is understood to relate to a preformed plastic and/or foil packaging used for small consumer product goods, foods and pharmaceuticals (chewing gum, etc.). The blister pack typically will provide a cavity or pocket created with a formable web or an aluminum foil or plastic on one side, and a paperboard or other less conformable back wall or seal on the other side. The product may be easily expelled and retrieved by a user by exerting force on the back wall to expel the product through the aluminum foil or plastic compartment, and into a users' hand, for example. In typical blister packs, the content of a blister pack "chamber" compartment will be released upon a user applying direct force against the back wall of the "pack", with the back wall remaining intact.

Generally, upon exerting a direct pressure to the back wall of the blister pack, the weakest part of the front wall or encasing layer of the blister "pack" assembly will be thrust out of the compartment, as the front wall material is perforated by the product contained in the "pack" compartment. When pressure is applied to the blister, the backing seal ruptures, and the contents of the blister are ejected through the tear in the backing seal.

Blister pack units are described in German patent application DE10343668A1, which relates to a leak-tight blister pack for medication. In another example, German patent application DE202007003050U1 relates to a blister pack for a liquid substance that is released into the external environment by diffusion.

The medical arts remain in need of improved devices, materials and methods for handling and safely managing the disposal of liquid or semi-liquid materials having blood or blood components, and that avoid and/or minimize user contact.

US 5,185,007 discloses a suction drainage control system wherein waste-treating material is released into a sealed chamber. The chamber comprises of a cover with a flexible liner sealed to and suspended therefrom. A normally closed reservoir is provided on an underside of the cover for storing waste-treating material and an externally operated actuator is provided on the cover to open the reservoir and release the waste-treating material within the sealed container.

WO 2021/054297 discloses a waste liquid receiving receptacle that receives a predetermined waste liquid sucked by a suction force of a suction source, a waste liquid reservoir that includes the waste liquid receiving receptacle, and a waste liquid suction system.

SUMMARY OF THE INVENTION

The present invention is defined in the appended set of claims.

The present disclosure provides a solution to these and other problems existing in the art.

In a general and overall sense, disclosed herein are materials, devices, and systems for safely managing and disposing of potentially health hazardous materials that may include a fluid component, such as those materials collected during or following a laboratory, medical or other procedure. In particular, devices and methods for processing and disposal of materials collected during a surgical procedure, that are at risk of containing blood or blood products, are presented. The disclosed devices and methods include a covering assembly, such as a lid, cover, and/or cap, that provides a tool which permits an enhanced safety, contact-free disposal solution for potentially biohazardous materials. In this sense, a waste collection and biohazard disposable system is provided.

The device, in one disclosure, comprises a covering and/or lid assembly comprising a first compartment containing a coagulant material capable of coagulating a blood component (red blood cells), the first compartment having a top side and a bottom side, wherein the top side comprises a compressible button or dimple. In this disclosure, the compressible button or dimple will be preferably situated above a frangible layer located on the bottom side of the first compartment, wherein the frangible layer, upon rupture, will release the coagulant material and function to coagulate and/or gelatinize blood components in a material, such as red blood cells.

In some disclosures, the coagulant material is selectively released from the compartment upon rupture of the frangible layer/covering, and into a container or other receptacle. Thus, in operation, when the compressible button or dimple is compressed, the coagulant, in particular a blood coagulant, will be selectively released. Any blood component in the container and/or compartment will thereafter quickly form a coagulated semi-solid, gel-like state, thus permitting an essentially spill-free, more easily disposable material solid and/or semi-solid mass.

In other disclosures, the comprises a covering and/or lid assembly comprising a second compartment containing a red blood cell flocculant material, capable of flocculating a blood component (red blood cells), the second compartment having a top side and a bottom side, wherein the top side comprises a compressible button or dimple. In this disclosure, the compressible button or dimple will be preferably situated above a frangible layer located on the bottom side of the compartment, wherein the frangible layer, upon rupture, will release the red blood cell flocculant material and function to flocculate red blood cells by associating with the surface of the red blood cells, thereby inhibiting blood coagulation.

Virtually any coagulant capable of coagulating blood, or more specifically, red blood cells, and even more specifically, flocculated red blood cells, is a material that may be used in the present devices/lids/caps.

Table 1 presents examples of human red blood cell (RBC) flocculants. RBC's that have been flocculated (not coagulated) with these flocculants, may be further coagulated to provide a solid or semi-solid mass, upon being exposed and/or combined with a coagulant as provided with the present devices and methods.

Table I

| Chemicals | Descriptions |
|---|---|
| Dextran 80 + CaCl$_2$ | Literature indicates that Dextran 80 plus a divalent cation like Ca$^{2+}$, and Ba$^{2+}$ will increase aggregation of RBCs |
| Polyethylenimine (PEI) with different molecular weight | PEI is a polymer composed of large number of positively charged amine groups, which is expected to attract RBCs to cause RBC settlement. |
| Polyacrylamide (PAM) with different molecular weight | PAM is widely used as a flocculants for water treatment. It can be configured to either positive or negative charge. Positively charged PAM is toxic to aquatic wildlife. |
| Polydiallyldimethylammonium chloride (PolyDADMAC) with different molecular weight | PolyDADMAC is a positively charged water-soluble polymer |

RBCs present in a fluid containing blood become bound to each other in the presence of an RBC flocculant, and in this manner form heavier particles that settle within the container/bottle/tube/collapsible bag or other vessel within which a sample/material is collected. Once the flocculated RBCs in a collected biological material settle, the flocculated settled RBCs may be easily re-dispersed, and do not coagulate and/or do not form a solid mass that may be easily and safely disposed of. Therefore, these may be coagulated by providing one or more appropriate coagulants to the flocculated settled or dispersed red blood cells.

In some disclosures, the flocculant comprises poly diallyl-dimethyl-ammonium chloride (PolyDADMAC). Virtually any type of flocculant may be used to achieve the sedimentation of red blood cells in a liquid. Examples of RBC flocculants include polymeric RBC flocculants and non-polymeric RBC flocculants. Non-polymeric RBC flocculants may include acids, such as HCl or other acid molecule.

Selection of an appropriate solidifying. jelling (or blood coagulant) requires that the agent be capable of forming a solid, jell-like or semi-solid mass from a liquid comprising red blood cells that have been flocculated.

Kits are provided wherein a blood solidifying, jelling agent is packaged as part of a lid/cap/cover assembly, and configured to securely attach (screw, snap, etc.) to a collection vessel opening, such as to the top opening of a collection vessel or device. In some disclosures, the cap/lid/cover lid assembly will be configured to include a blister pack as described above, and will contain the blood solidifying/jelling agent within the blister pack as part of a lid assembly. The lid assembly, in some disclosures, will be configured so as to attach securely to an opening of a collection vessel.

In some disclosures, the blister pack may comprises two films which in turn may be made up of several layers of different or identical materials. The films may be made of plastics and/or metal, e.g. aluminum, while other materials such as paper or the like may theoretically also be used, or used in addition. The two films are, in particular, a base layer or base film (support) and a cover layer or cover film (cover).

In the base film or support there may be one or more wells or depressions for holding the liquid, into which the liquid, particularly a pharmaceutical formulation, is introduced. The cover film or the cover is then placed on the base film or support and fixed or attached thereto, and this is done, for example, at the edges or in a connecting region, particularly in flatly abutting surface regions, preferably by adhesion, heat-sealing, welding or the like.

The blister pack may be sealed by an inert compound that minimally interferes with analysis of the blood, blood components, or other chemicals to be introduced into a collection canister. In order to allow the blister to rupture under pressure, a frangible seal is often provided around at least part of a perimeter of the blister cavity. Such frangible seal technologies allow relatively simple and controlled release of reagents, whilst eliminating the need for complex fluid handling systems or external piercing means.

The sealing material for the blister pack may be a plastic film made from a material comprising, for example, polyethylene, polypropylene, polybutylene, polyvinyl chloride, or a combination thereof. The sealing material for the blister pack may also be aluminum foil.

The blister cavity or pocket may be made from a formable web, usually a thermoformed plastic such as PVC, PVDC, Polychlorotrifluoroethylene (PCTFE), or cyclic olefin copolymers (COC) or polymers (COP). These aforementioned materials may be combined with polypropylene (PP), polyethylene (PE), or glycol-modified polyethylene terephthalate (PETg) to add more protection.

Blood Solidifying/Jelling Agents: Various types of blood solidifying/jelling agents may be used.. These may include cellulosic materials, salts thereof, salts of inorganic compounds such as Cu (II), Ag (I), Fe (II), Fe (III), Ti (IV), and Ni (II). The solidifying/jelling agent may be aerosolized, and/or applied in a powder form, of a cellulose rich compound. By way of example, Liqui-Loc®, PolySorb™, a calcium sodium salt of micro-dispersed oxidized cellulose (QUICKSTOP!®) or similar agent may be employed to solidify a composition for disposal. An effective solidifier/jelling agent may comprise a solution containing any of the following compounds: methanol; poly-aluminum chloride; silver nitrate; copper (II) sulfate, copper (II) bromide; ferric sulfate; ferric sub-sulfate; ferric chloride; aluminum sulfate; or zinc sulfate. The flocculated red blood cell solidification and/or jelling agent may be provided in the form of a powder, a tablet, or a liquid as part of the present devices and methods employing the devices. Table II provides examples of the flocculated red blood cell solidifying and/or jelling agents. As a component of the lid/cap assembly, a powder form of the cellulosic material may be provided in a compartment of the lid assembly, and easily released into the container/canister, without risk of exposure of the container contents to the operator/technician.

| **Chemicals** | Chemicals |
|---|---|
| Carboxymethylcellulose Sodium  Carboxymethylcellulose Sodium Salt  Carboxymethyl Ether Cellulose | Carboxymethylcellulose Sodium; carboxymethylcellulose Sodium Salt' Cellulose Gum Carmellose Sodium ($C_8H_{16}NaO_8$, MW: 263.2 g/mol)  Sodium;2,3,4,5,6-pentahydroxyhexanal;acetate ($C_8H_{15}NaO_8$, MW: 262.2 g/mol) |
| aluminum chloride  styptic powder | MediVac™, MediVac Solid Plus™  StatSorb™ |
| copper (II) sulfate | LiquiLoc Plus™ |

(continued)

| Chemicals | Chemicals |
|---|---|
| alum | Body Fluids Solidifier™ |
| potassium aluminum | Red Z™ Solidifier Control |
| potassium ferrate | Isosorb™ Liquid Treatment System |
| ferric or ferrous sulfate | zinc sulfate |
| ferric subsulfate | methanol |
| ferric chloride | aluminum sulfate (Alum) |

Covering/Lid Assembly for Canisters, Collection Bags and Containers: Collection devices including canisters, collection bags and containers suitable for the collection and visual estimation of blood loss may be used with the covering and/or lid assemblies provided herein.

In some disclosures, the lid/covering assembly may include one or more than one frangible compartments. One of the frangible compartments will comprise a coagulant that will effectively coagulate flocculated red blood cells in a fluid.

In another disclosure, the lid/covering assembly will comprise a first frangible compartment comprising a cellulosic solidifier/jelling agent that will effectively solidify and/or remove liquid components of a preparation of suspended flocculated red blood cells in a fluid, and a second frangible compartment comprising a red blood cell flocculant. This disclosure of the lid/covering assembly is envisioned for use with a collection canister or other container that has an interior that has not been treated to include a red blood cell flocculant, such as a coating of a red blood cell flocculant (e.g., PolyDADMAC). As part of a blood loss assessment device, the lid having these two frangible compartments will be employed together with a collection device that has not been previously treated to include an interior surface comprising a coating of a red blood cell flocculant. Prior to or during collection of a fluid into the container, the red blood cell flocculant in the second frangible compartment will be released into the interior of the collection container. The presence of the red blood cell flocculant will allow any red blood cells in the collected material /fluid to settle in the container, and form a discernable settled red blood cell level. However, the settled flocculated red blood cells may be readily dispersed if the contents of the container are disturbed, and there is no solid coagulation. In order to transform the flocculated red blood cells into a more easily disposable solid and/or semi-solid mass, the flocculated red blood cell solidifying and/or jelling material contained in the second frangible compartment of the lid assembly may be securely and simply released into the interior of the container, without opening the container or removing the lid/covering assembly. A safe, secure, contact-free assembly and device is provided for both assessing settled red blood cell levels (and calculating blood volume therefrom) in a material, and disposing of the collected material having or suspected to contain blood and/or red blood cells, in a single assembly, without exposing the contents to the environment or the user.

The lid assembly in either disclosure may be configured to have a circumference that permits the lid assembly to securely and snugly fit over the top or opening of a collection device, such as a biological fluid collection device. The biological fluid collection device may take the form of such devices as those described in U.S. Patent 10,401,347 and U.S. Patent Application Numbers 16/363,674, 16/257,876, and 16/257,673, and corresponding patents and patent applications outside of the United States, that include as a component a red blood cell flocculant provided on a surface of the collection container, such as a surface coating of a red blood cell flocculant on the inside surface of a container. The container may be in the shape of a canister, an envelope or bag, or other configuration onto which a coating of a red blood cell flocculant material may be provided.

In summary, the present covering and/or lid assembly may be used together with a collection device, such as a canister or collection bag or sleeve, that is preferably sufficiently transparent or at least opaque so as to permit the visual detection of a level of material, such as a level of sediment, flocculated red blood cells (settled RBCs, not coagulated blood) within the collection device. The collection device can be of a solid or flexible material, such as a hard plastic or glass, or a flexible material, such as a plastic or other non-rigid material suitable for containing a liquid and/or semi-liquid state material comprising or suspected to comprise blood. Such liquid and/or semi-liquid material comprising blood or suspected to comprise blood, includes a liquid medical waste material comprising blood.

These descriptions are provided by way of example and are not intended to provide limitation of potential disclosures envisioned for use and practice of the present devices.

BRIEF DESCRIPTION OF THE DRAWINGS

Figure 1 illustrates a disclosure of the covering and/or lid assembly disclosed herein.

Figure 2 illustrates a disclosure of the collection system disclosed herein.
Figure 3 illustrates a plan view of one disclosure of the covering assembly and/or lid top portion disclosed herein.
Figure 4 illustrates a plan view of one disclosure of the covering assembly and/or lid bottom portion disclosed herein.
Figure 5 illustrates another disclosure of the covering assembly disclosed herein.
Figure 6 illustrates a plan view of another disclosure of the covering assembly top portion disclosed herein.
Figure 7 illustrates a plan view of another disclosure of the covering assembly bottom portion disclosed herein.

DETAILED DESCRIPTION

Unless defined otherwise, all technical and scientific terms used herein have the same meaning as commonly understood by one of skill in the art to which the disclosure pertains.

As used here, the term "flocculant" is intended to mean a molecule that has a cationic charge that is capable of facilitating the coalescence of RBCs in a fluid at room temperature, and form a settled RBC mass with less than 30 minutes at room temperature without centrifugation. A flocculant as defined herein does not provide for coagulation , solidification or jelling of a liquid comprising blood or a blood component (e.g., red blood cells).

Reference to an element by the indefinite article "a" or "an" does not exclude the possibility that more than one element is present, unless the context clearly requires that there be one and only one element. The indefinite article "a" or "an" thus usually means "at least one."

As used herein, "patient" or "subject" means an individual having symptoms of, or at risk for, cancer or other malignancy. A patient may be human or non-human and may include, for example, animal such a horse, dog, cow, pig or other animal. Likewise, a patient or subject may include a human patient including adults or juveniles (e.g., children). Moreover, a patient or subject may mean any living organism, preferably a mammal (e.g., human or non-human) from whom a blood volume is desired to be determined and/or monitored from the administration of compositions contemplated herein.

As used herein, "waste" means a mixture that incudes blood or a blood component, such as red blood cells. Bio-waste from hospital/clinical sources are examples.

As used herein, "blood solidifying and/or jelling enhancing substance" or "blood coagulating material" means a substance that may provide a solid ,semi-solid of gelatinous mass from a liquid or semi-liquid material comprising blood or a blood component, such as red blood cells.

As used herein, "about" means within a statistically meaningful range of a value or values such as a stated concentration, length, molecular weight, pH, sequence identity, timeframe, temperature or volume. Such a value or range can be within an order of magnitude, typically within 20%, more typically within 10%, and even more typically within 5% of a given value or range. The allowable variation encompassed by "about" will depend upon the particular system under study, and can be readily appreciated by one of skill in the art.

The following examples are presented to demonstrate some disclosures of the invention.

Example 1 - Canister Covering/Lid Assembly

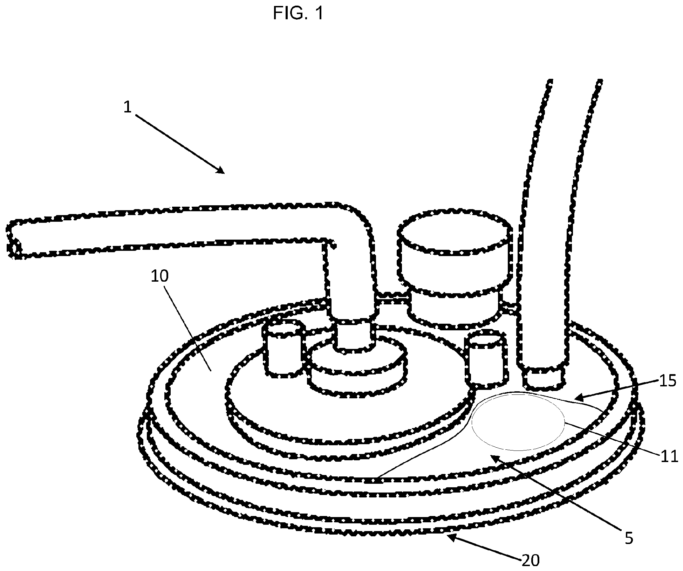

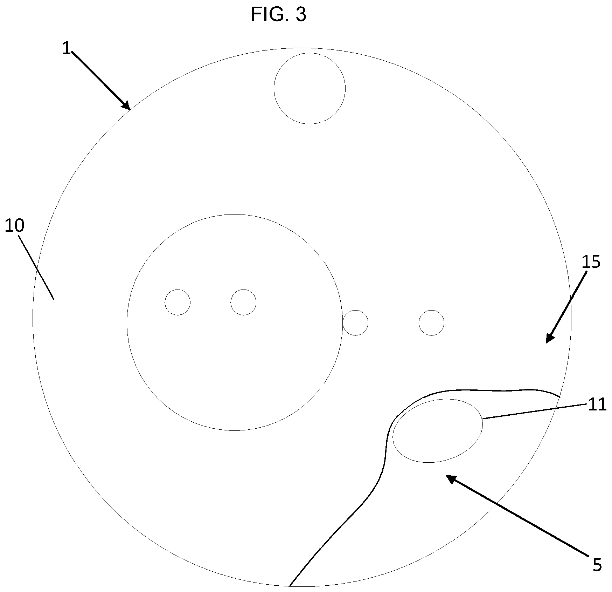

The present example presents a description of one disclosure of the covering assembly/lid 1. The covering assembly/lid is illustrated in Figures 1, 3, and 4.

The covering assembly/lid 1 includes a top portion 10 and a bottom portion 20 , and further includes a frangible compartment 5 situated on a first surface 25 of the covering assembly bottom portion 20. Covering assembly/lid 1 may comprise a transparent plastic material, such as a polyethylene or a polypropylene. The frangible compartment 5 contains a blood coagulation enhancing substance 50 that is selectively releasable from the frangible compartment 5.

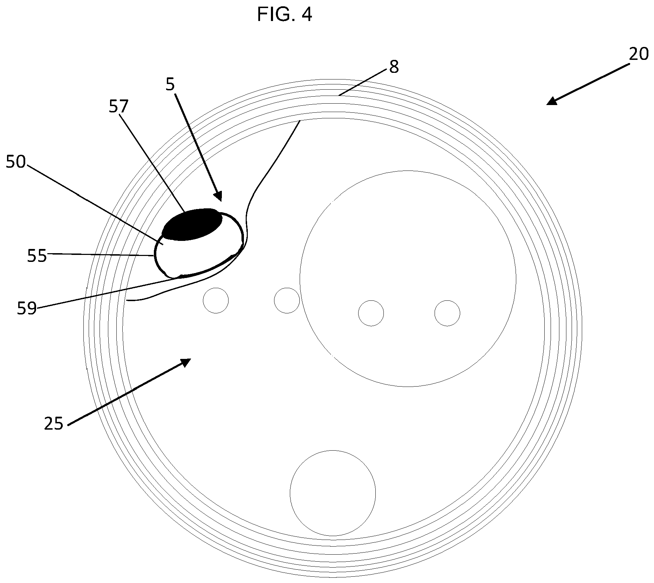

In one disclosure, the blood coagulation enhancing substance 50 may be powder, as illustrated in Figure 4. However, the blood solidifying and/or jelling enhancing substance and/or biological waste solidifier 50 may be in the form of a powder, a tablet, or a liquid, and may be selected from any of the aforementioned blood solidifying and/or jellifying compounds previously listed. The blood solidifying and/or jellifying enhancing substance and/or biological waste solidifier 50 may be enclosed in a frangible compartment 5. The frangible compartment may be provided as a blister pack, the blister pack comprising a release side comprising a frangible material, such as an aluminum, tin, or other foil, aplastic, or other frangible material. The blood solidifying and/or jellifying enhancing substance 50 may be contained within the blister pack. Alternatively, the blood solidifying and/or jellifying enhancing substance and/or solidifier may be packaged in a box, a bottle, a tray, a cartridge, or a card.

Covering assembly/lid 1 may also include a coupling adapter 8 along a perimeter 12 of the covering assembly 1. The coupling adapter 8 with a threaded connection that is suitable for attaching the covering assembly 1 onto a canister 30 to provide a fluid-tight seal. Coupling adapter 8 may comprise, for example, a threaded connection, or a screw connection, or a locking connection. As shown in Figure 1, the covering assembly/lid 1 may include a compressible button or dimple 11, that is comprised of a non-frangible material, on a first surface 15 of the covering assembly top portion 10. The compressible button or dimple 11 in some disclosures is situated above the frangible compartment 5, and when a compressive force (such as pushing the dimple/button "in"), is applied by a user to the compressible button or dimple 11, the blood coagulation enhancing substance 50 is released from the delivery compartment 55, as the frangible layer of the compartment opens/breaks, for example, by the force exerted by a substance contained within the frangible compartment against the frangible layer and/or the pressure exerted upon pressing the button or dimple 11.

In one disclosure and as shown in Figure 4, a first frangible compartment 55 comprises a non-frangible top layer 57 and a bottom frangible layer 59. Application of pressure to the non-frangible layer 57 results in force being applied to bottom frangible layer 59, causing the frangible layer to break or tear. The break and/or tear of the frangible layer in turn results in the release of the blood coagulation enhancing substance 50, and into an enclosed collection device. The compressible button and/or dimple 11 located on the non-frangible top layer 57 may be comprised of one or more plastic materials that are pliable and able to be bent by an applied pressure. The bottom frangible layer 59 may comprise a layer of aluminum film or other material that will be broken/torn by an applied pressure to the button and/or dimple. The applied pressure may originate from, for example, a user pressing the button and/or dimple in or down, thus creating air pressure to be applied against the interior of the frangible compartment and through the bottom frangible layer 59.

Example 2 - Collection System

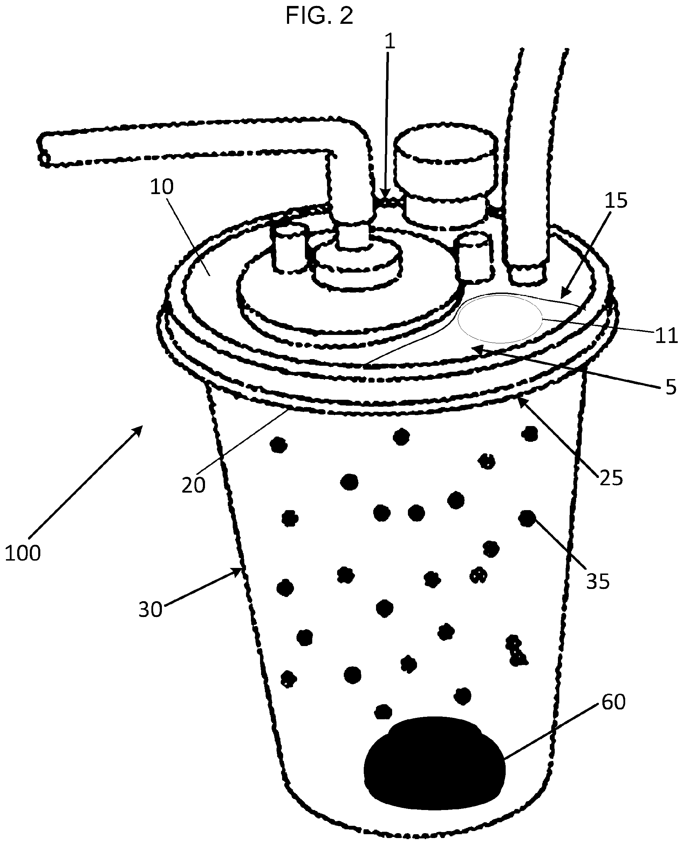

The present example presents the collection system 100. The system is illustrated in Figure 2. The collection system 100 includes a collection vessel that may connect to a covering and/or lid assembly 1. The covering and/or lid assembly 1 includes a top portion 10 and a bottom portion 20, and further includes a frangible chamber 5 situated on a first surface 25 of the covering assembly bottom portion 20.

Frangible chamber 5 contains a blood solidifying/jelling agent and/or blood coagulation enhancing substance 50 that is selectively releasable from the frangible chamber 5. The blood solidifying and/or jellification enhancing substance 50 may be enclosed in a compartment 55, which may be a blister pack. The blister pack is made up on one surface a frangible material, such as a surface comprising plastic and/or an aluminum foil material. The covering and/or lid assembly 1 has a coupling adapter 8 along a perimeter 12 of the covering assembly 1, and the coupling adapter 8 with a threaded configuration that is suitable for attaching the covering assembly 1 onto the top opening of a canister 30 to provide a secure, fluid-tight closing.

Covering assembly 1 may include a compressible button or dimple 11 on a first surface 15 of the covering assembly top portion 10. The compressible button or dimple 11 may be situated above the detachable chamber 5. When the compressible button or dimple 11 is compressed, the button or dimple 11 pushes air that engages the frangible compartment 55, which causes the blood solidification and/or jellification enhancing substance 50 within the delivery compartment 55 to be released into the adjacent canister 30. Canister 30 a fluid sample 60 including fluids such as settled RBCs and other media to be disposed. When the blood solidifying and/or jellification enhancing substance 50 released from delivery compartment 55 and is mixed with the fluid sample 60 in canister 30, the waste components of fluid sample 60 will solidify and/or jell (or otherwise coagulate), preventing the accidental spill or sloshing of a biohazard material onto technical personnel. Jelling/solidification of the fluid sample 60 by the blood solidifying/jelling (or coagulation) substance 50 may then take place, of the flocculated RBCs, in the presence of flocculant 35. The solidifying/jelling agent will comprise a composition comprising a highly absorbent cellulosic material as cellulose. Such a cellulosic material may comprise sodium; 2,3,4,5,6-pentahydroxyhexanal; acetate. A powdered form of this or other appropriate agent may be included in a compartment of the lid/cap assembly.

Waste is collected in collection system 100 a single time and then the entire system and waste contents are discarded. Collection system 100 is self-contained, does not require cleanup for disposal of the contents therein, and thereby prevents creation of a biohazard. No additional solution or other cleaning is required for disposal of the contents in collection system 100.

Collection system 100 may be propped up or set up on a stand, such as a tower for IV drip containers. Each time a medical procedure is completed and fluids are collected in the collection system 100, the enclosed system and the contents therein are disposed. A new collection system is then substituted in place of the used collection system, and the new system is used to process additional waste. The canister 30 may be, for example, a flocculated container, having flocculant 35. Examples of flocculated containers are described in U.S. Patent Application Numbers 16/363,674, 16/257,876, 16/257,673, and 15/868,983.

Collection system 100 also provides for estimation of blood volume in the contents within collection vessel 30.

Example 3 - Contact-Free Biological Material Collection and Disposal System

The present example presents a contact-free system for collecting and disposing of biologically hazardous materials that minimizes user handling. The system provides for collection and processing of a fluid suspected to include or including a biological material, such as blood, into a collection canister, assessing a volume of blood that is present in the collected material, and transforming the content of the container into a readily disposable solid and/or semi-solid mass in the container, without removal of the covering/lid assembly.

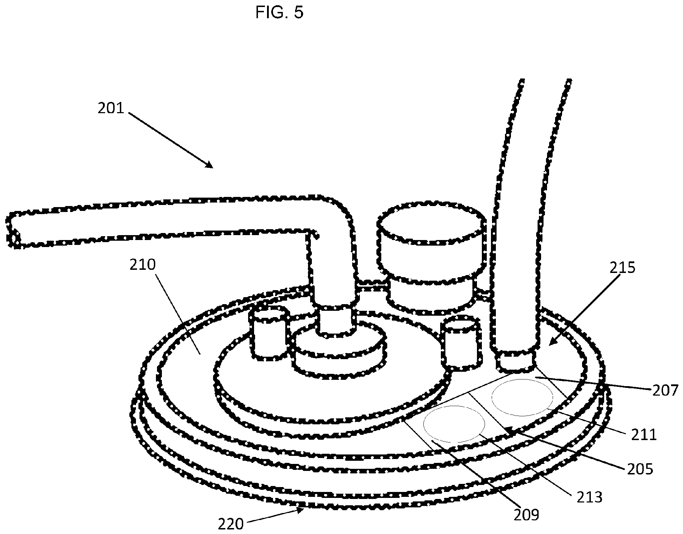

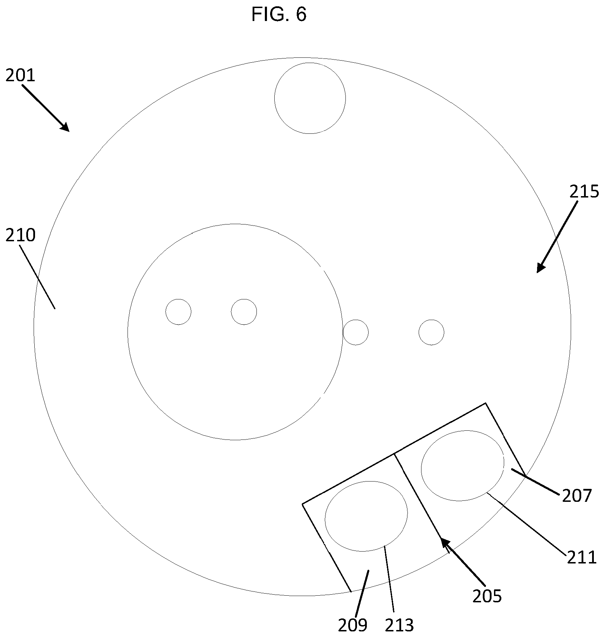

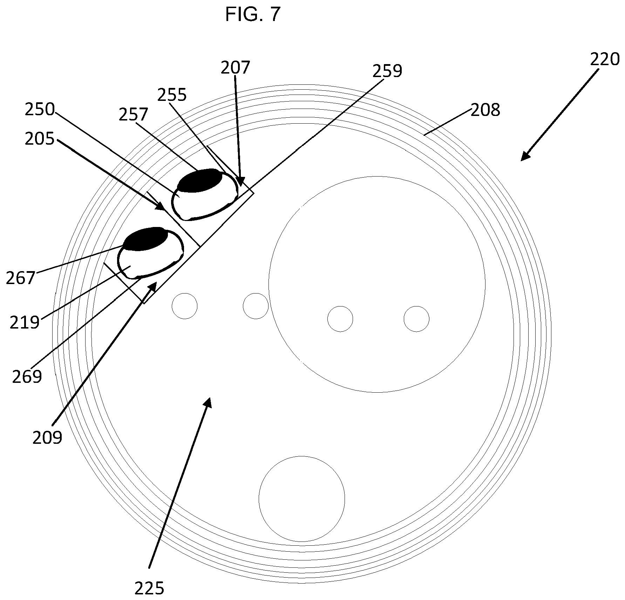

A disclosure of the covering assembly is illustrated in Figures 5, 6, and 7. Covering assembly 201 includes a top portion 210 and a bottom portion 220. A first chamber includes a frangible material layer 205 situated on a first surface 225 of the covering assembly bottom portion 220. The covering assembly and/or lid 201 may comprise a plastic material, e.g., polyethylene or polypropylene.

The frangible material layer 205 will be positioned at a bottom side of a first compartment 207. The first compartment may contain a red blood cell flocculant material or a blood coagulant material 250. A first button/dimple 211 will be located directly above the frangible chamber, and upon pressing the button and/or dimple, the material in the compartment will apply a force against the frangible material surface that breaks the surface, thus expelling the material into an interior chamber of a collection container. Thus, as the surface of the first frangible chamber is breached from the application of the force, the material is released into the container where it will mix and contact with the fluid contents.

A second compartment 209 may also be provided, this compartment comprising a preferred material, such as a red blood cell flocculant or blood coagulant (capable of transforming flocculated red blood cells into a solid and/or semi-solid mass or gel). This solid and/or semi-solid mass may then be easily disposed of, without risk of splashing or spilling onto another person, technical personnel, or on/in the surrounding area.

A second button/dimple 213 defining one side (the top side) of the second compartment, will be located directly above a second frangible layer, and upon pressing the second button and/or dimple, the material within the second compartment (such as a red blood cell flocculant or a flocculated red blood cell coagulant material) will be forced through the frangible layer of the compartment and into a collection device interior.

The first button/dimple 211 and the second button/dimple 213 may each be substantially level with first surface 215 of the covering assembly top portion 210 to improve the compactness of covering assembly 201, and allow for stackable storage for more than one covering assembly 201. This may also ensure that the first button/dimple 211 and the second button/dimple 213 do not become exposed to force that would press upon them upon packaging and shipping of the covering assembly 201.

The flocculated red blood cell solidifying/jelling and/or coagulant substance may be a powder, or other form, such as a tablet, or a liquid. The flocculated red blood cell solidifying/jelling and/or blood coagulant, red blood cell flocculant, or both, may be enclosed in a blister pack within the first compartment or the second compartment, for example, within a pack made of a plastic and/or aluminum foil. The flocculated red blood cell solidifying/jelling and/or red blood cell coagulant, or red blood cell flocculant may alternatively be packaged in another material such as, for example, a box, a bottle, a tray, a cartridge, or a card, within the first compartment or second compartment, or both compartments.

Covering assembly 201 also includes a coupling adapter 208 along a perimeter 212 of the covering assembly 201, and the coupling adapter 208 with a threaded connection that is suitable for attaching the covering assembly 201 onto a canister 230 to provide a fluid-tight seal. Coupling adapter 208 may comprise a threaded connection, or a screw connection, or a locking connection.

Covering assembly 201 may be interchangeably used with the collection system 100.

Example 4 - Blood Volume in a Liquid - Calculation Blood (containing no Anticoagulant), High MW Flocculant

A high molecular weight flocculant, established by the present study to effectively flocculate human red blood cells in human blood, is Poly (diallyldimethylammonium chloride). The high molecular weight form of PolyDADMAC material has an average molecular weight of 400,000-500,000. Lower molecular weight forms of PolyDADMAC may also be used. This flocculant in a powder, tablet, liquid or other form may be included in a compartment of the lid and/or cap of the present device, and then selectively released into a canister on which the lid/cap is fitted.

As part of the claimed blood management systems, the amount of blood in a collected material in a canister may be calculated according to the following formula:

$$V_m / (Hct \times \acute{\eta}) = V_b$$

Where $V_b$ is equal to the calculated volume of blood in a material, $V_m$ is equal to the observed sedimentation level of red blood cells in a container having a flocculant, $\acute{\eta}$ is equal to the packing ratio determined for the container/container and *Hct* is equal to the hematocrit of the animal/human from whom the liquid material was collected.

Example 5 - Polymeric solidifying materials with Flocculated Red Blood Cells Sedimented from Human Blood (No Anticoagulant)

The present example illustrates the use of the present canister covering assembly including a blood coagulant/-

blood component solidifying and/or jelling agent in a compartment for processing flocculated red blood cells from a volume of human blood (no anti-coagulant). The human blood employed in the following studies did not contain an anti-coagulant. The solidifying/coagulating agent examined was a super absorbent cellulosic material of high molecular weight cellulose-containing material (by way of example, Liqui-Loc Plus ™ Solidifier). At the end of the 30 minute sedimentation period, 1 oz. (about 100 ml.) of the cellulosic solidifier (Liqui-Loc Plus™) was added to the Test 4 canister. The settled RBCs and other material in the container (saline, other blood components) were not in a solid and/or gel-like condition at the 30 minute time period prior to adding the solidifier/jelling agent. In order to ascertain in the solidifier would be effective for solidifying the sedimented flocculated RBCs and other material in the canister, the solidifier and/or jelling agent was released into the canister. The material in the canister, including the flocculated RBCs, began to solidify/jell within about 1 minute the entire contents of the canister had formed a solid, gel-like mass (a "plug"). This is ready to dispose of safely as a biohazard material that handling contains exposure. 2 minutes, thus, the solidified, gel-like material in the canister was suitable for safe and efficient disposal without risk of spill or contact with persons handling the container. Thus, as part of the presented canister covering/lid, a coagulant and/or solidifying/jelling agent may be released into the canister from the compartment on the lid by compressing the dimple located above the compartment, to easily and quickly solidify/jellify the contents for easy and safe disposal of biohazard waste, in compliance with conventional hazardous waste handling practice at hospital/facility and/or medical waste collecting facilities.

| Test 1 - Human Adult Male; Hct. 45%; | | |
|---|---|---|
| Mixture: 131 mL Blood, 500 mL Saline | | |
| Time (min) | Volume | Subject: Human Male Adult<br>Canister Type: 1200 ml, floc coated= PolyDADMAC |
| 0 | | |
| 1 | 50 | Adult Male Hematocrit: 45 % |
| 2 | 60 | Total Volume: 631 |
| 3 | 65 | Settled RBC at 20 min., RT: Vm=75mL @T:20 |
| 4 | 70 | |
| 5 | 75 | |
| 6 | 75 | Conc: 20.7 % Blood/Saline |
| 7 | 75 | |
| 8 | 75 | Calculated Blood Vol ($V_B$) 75/(.45 x 1.2) = <u>138.9</u> |
| 9 | 75 | |
| 10 | 75 | 10% +/- Range=<u>118 to 143 mL</u> |
| 11 | 75 | |
| 12 | 75 | No solidifying/gelling agent-Material remained in liquid state at 30 min. |
| 13 | 75 | |
| 14 | 75 | |
| 15 | 75 | |
| 20 | 75 | |
| 30 | 75 | |
| 60 | 75 | |

| Test 3 - Human Adult Female; Hct. 40% | | |
|---|---|---|
| Mixture: 150 mL Blood, 225 mL Saline | | |
| Time (min) | Volume | Subject: Human Adult Female |
| 0 | | Canister Type: 1200 ml |
| 1 | 100 | Floc coated= polyDADMAC |

(continued)

| Test 3 - Human Adult Female; Hct. 40% | | |
|---|---|---|
| Mixture: 150 mL Blood, 225 mL Saline | | |
| 2 | 50 | Adult Female Hematocrit: 40 |
| 3 | 60 | |
| 4 | 70 | Total Volume: 350 |
| 5 | 70 | |
| 6 | 75 | Settled RBC at 20 min. , RT: Vm=75 mL @T:20 |
| 7 | 75 | |
| 8 | 75 | Conc: 40 % Blood in Saline |
| 9 | 75 | |
| 10 | 75 | |
| 11 | 75 | Calculated Blood Vol ($V_B$) 75/(.40 x 1.2) = 138.9 ml. |
| 12 | 75 | |
| 13 | 75 | 10% =/1 Range=135 to 165 mL |
| 14 | 75 | |
| 15 | 75 | |
| 20 | 75 | |
| 30 | 75 | No solidifying/gelling agent-Material remained in liquid state at 30 min. |
| 60 | 75 | |

| Test 2 - Human Adult Female; Hct. 40% | | |
|---|---|---|
| Mixture: 170 mL Blood, 400 mL Saline | | |
| Time (min) | Volume | Subject: Human Adult Female |
| 0 | | Canister Type: 1200 ml. |
| 1 | 110 | Floc coated=polyDADMAC |
| 2 | 100 | Adult Female Hematocrit: 40 % |
| 3 | 100 | |
| 4 | 100 | Total Volume: 570 mL |
| 5 | 95 | |
| 6 | 90 | Settled RBC at 20 min., RT: Vm=90mL @T:20 |
| 7 | 90 | |
| 8 | 90 | |
| 9 | 90 | Conc: 30 % Blood in Saline |
| 10 | 90 | |
| 11 | 90 | Calculated Blood Vol. ($V_B$) 90/(.40 x 1.2)=166.7 mL |
| 12 | 90 | |
| 13 | 90 | |
| 14 | 90 | 10% +/- Range=153 to 187 mL |
| 15 | 90 | |

(continued)

| Test 2 - Human Adult Female; Hct. 40% | | |
|---|---|---|
| Mixture: 170 mL Blood, 400 mL Saline | | |
| 20 | 90 | No solidifying/gelling agent-Material remained in liquid state at 30 min |
| 30 | 90 | |
| 60 | 90 | |

| Test 4- Human Adult Male | | |
|---|---|---|
| Mixture: 200 mL Blood, 150 mL Saline | | |
| Time (min) | Volume | Subject: Human Adult Male<br>Canister Type: 1200 ml Floc PolyDADMAC coated |
| 0 | | |
| 1 | 300 | Adult Male Hematocrit: 45 |
| 2 | 300 | Total Volume: 350 |
| 3 | 250 | Settled RBC at 20 min., RT: Vm=115mL @T:20 |
| 4 | 225 | |
| 5 | 200 | |
| 6 | 175 | Conc: 57 % Blood in Saline |
| 7 | 175 | |
| 8 | 150 | Calculated Blood Volume ($V_B$) 115/(.45 x 1.2) = 212.9 mL. |
| 9 | 140 | |
| 10 | 125 | |
| 11 | 120 | 10% =/- Range=180 to 220 mL |
| 12 | 115 | |
| 13 | 115 | Flocculant, sedimented, Solidifying Agent: Cellulose composition (Liqui-Loc Plus™, 1 ounce), Carboxycellulose |
| 14 | 115 | |
| 15 | 115 | |
| 20 | 115 | |
| 30 | 115 | |
| 60 | 115 | |

In Test 4, the sedimented RBCs at the end of the 30 minute sedimentation period in the canister were stirred to disrupted and RBCs, making them to become suspended in the liquid. A cellulosic solidifying agent (for example, Liqui-Loc Plus™, Carboxycellose) was added to the suspension (1 oz. Liqui-Loc, Carboxycellose). Within about 1 minute, the mixture began to solidify, and at 2 minutes the entire contents of the canister formed a solidified, jell state. This jelled/solidified material was in this state a safe and secure form for bio-hazard waste safe disposal, and eliminated any contact of the canister contents with personnel

Example 6 - Solidifying and/or Coagulating Polymeric Materials suitable for Solidifying Biological Waste Products with Flocculated Human RBCs

Blood components, such as red blood cells, sedimented from blood that had not been processed to include an anti-coagulant, will upon contact with a flocculant, yield red blood cells that are flocculated and that will form a uniform, observable sedimentation level. Human red blood cells exposed to a high molecular weight flocculant in a container (e.g., high mw PolyDADMAC), will form an observable sedimentation level. For disposal purposes in accord with accepted biohazard safe hospital/medical facility protocols, liquid or semi-liquid hazardous bio-waste should be treated such as to

## EP 4 185 345 B1

provide a solid, semi-solid, and/or jellified composition that will not splash, spill, or otherwise expose technicians and others to the canister contents, for safe disposal.

The canister covering assembly may include a compartment that includes a flocculated red blood cell solidifier. The solidifier (RBC coagulant, jelling agent) may comprise a super absorbent cellulose composition. Compositions employed in the health care industry (hospitals) for solidifying/jelling biological waste include several compositions that include cellulose (e.g., Liqui-Loc Plus, StatSorb, Isosorb, Medi Vac Solid Plus), and other highly absorbent formula components as part of an appropriate solidifying/jelling formulation.

It is to be understood that the disclosure is not limited to particular methods or systems, which can, of course, vary. It is also to be understood that the terminology used herein is for the purpose of describing particular disclosures only, and is not intended to be limiting.

A number of disclosures have been described. Nevertheless, it will be understood that various modifications may be made without departing from the scope of the present disclosure. However, the invention is defined by the appended claims.

BIBLIOGRAPHY

1. Nouri, S., et al., (2015). "Efficacy and Safety of Aluminum Chloride in Controlling External Hemorrhage: An Animal Model Study". Iran Red Crescent Medical Journal. 2015 Mar., 17(3).

2. Ratermann, A., et al., (1980), Journal of Agricultural Food Chemistry. 1980, 28, 2, 438-44.

3. U.S. Patent Application No. 12/924,547.

4. U.S. Patent Application No. 15/868,983.

5. U.S. Patent Application No. 16/363,674.

6. U.S. Patent Application No. 16/257,876.

7. U.S. Patent Application No. 16/257,673.

8. U.S. Patent 10,401,347.

9. German patent application DE10343668A1.

10. German patent application DE202007003050U1.

**Claims**

1.   A lid assembly (201) for attachment to a container, comprising:

a first compartment (207), a first compressible button (211), and a frangible covering (259), wherein the first compressible button (211) and frangible covering (259) define the first compartment (207), wherein the frangible covering is situated below the first compartment and a blood coagulating material (250) is contained within the first compartment (207),

wherein, when the lid assembly (201) is attached to a container, the blood coagulating material (250) is configured to be selectively released from the first compartment (207) into the container upon compression of the first compressible button (211), and

wherein the blood coagulating material (250) is configured to form a mass of coagulated blood within the container;

wherein the lid assembly (201) is **characterised in that** it further comprises:

a second compartment (209), a second compressible button (213), and a frangible covering (269), wherein the frangible covering (269) is situated below the second compartment (209) and a red blood cell flocculant (219) is contained within the second compartment (209),

wherein, when the lid assembly (201) is attached to a container, the red blood cell flocculant (219) is configured to be selectively released from the second compartment (209) into the container upon compression of the second compressible button (213), and wherein the red blood cell flocculant (219) is configured to form flocculated red blood cells within the container.

2. The lid assembly of claim 1, wherein the blood coagulant material comprises aluminum chloride, styptic powder, copper sulfate, aluminum sulfate, potassium aluminum, potassium ferrate, ferric sulfate, ferrous sulfate, ferric chloride, or zinc sulfate.

3. The lid assembly of claim 1, wherein the lid assembly is for attachment to the container, which is a collection canister.

4. The lid assembly of claim 1, wherein the red blood cell flocculant is polyDADMAC.

5. The lid assembly of claim 1, wherein the frangible covering (259, 269) comprises a foil or plastic film.

6. The lid assembly of claim 1, further comprising a coupling adaptor (208) along a perimeter of the lid assembly, the coupling adaptor comprising a threaded configuration configured to allow the attachment of the lid assembly to a top opening of a container.

7. The lid assembly of claim 1,

wherein said first compartment (207) comprises a first chamber and the blood coagulant material (250) is contained in the first chamber and the blood coagulant material (250) is configured to form a coagulated mass of blood; and

wherein said second compartment (209) comprises a second chamber, and the second chamber contains a red blood cell flocculant (219), and the red blood cell flocculant is configured to form flocculated red blood.

8. The combination of the lid assembly of claim 1 with a collection container (30), wherein the lid assembly (201) is attached to the collection container and the container contains blood; and wherein

the release of the blood coagulation (250) from the first chamber into the container upon compression of the button enables the formation of a coagulated mass of blood in the container to provide spill-free disposal of blood; and

the release of the red blood cell flocculant (219) from the second chamber into the container upon compression of the button enables the formation of flocculated red blood cells in the container, wherein said red blood cell flocculant (219) does not form a coagulated mass upon contact with blood in the container.

9. The combination of claim 8 wherein the coagulated mass is a solid mass.

10. The combination according to claim 8,

wherein the collection container has a canister configuration and a volume capacity of 100 ml, 250 ml., 500 ml, 1,200 ml, or 5,000 ml; and

wherein the lid assembly comprises a coupling adapter (208) suitable for securely attaching to an opening of the collection container.

11. The combination of claim 10, wherein the collection container has a volume of about 1200 ml. or 5000 ml.

12. The combination of claim 10, wherein the canister further comprises a surface coating of a red blood cell flocculant, and wherein the red blood cell flocculant is configured to form flocculated red blood cells within the collection container.

13. The combination of claim 12 wherein the red blood cell flocculant comprises polyDADMAC.

**Patentansprüche**

1. Deckelanordnung (201) zur Befestigung an einem Behälter, umfassend:

ein erstes Fach (207), einen ersten eindrückbaren Knopf (211) und eine zerbrechliche Abdeckung (259), wobei der erste eindrückbare Knopf (211) und die zerbrechliche Abdeckung (259) das erste Fach (207) definieren, wobei sich die zerbrechliche Abdeckung unterhalb des ersten Fachs befindet und ein blutgerinnendes Material (250) innerhalb des ersten Fachs (207) enthalten ist,

wobei, wenn die Deckelanordnung (201) an einem Behälter befestigt ist, das blutgerinnende Material (250) so konfiguriert ist, dass es bei Eindrücken des ersten eindrückbaren Knopfes (211) selektiv aus dem ersten Fach (207) in den Behälter hinein freigesetzt wird, und

wobei das blutgerinnende Material (250) so konfiguriert ist, dass es eine Masse aus geronnenem Blut innerhalb des Behälters bildet;

wobei die Deckelanordnung (201) **dadurch gekennzeichnet ist, dass** sie weiter Folgendes umfasst:

ein zweites Fach (209), einen zweiten eindrückbaren Knopf (213) und eine zerbrechliche Abdeckung (269), wobei sich die zerbrechliche Abdeckung (269) unterhalb des zweiten Fachs (209) befindet und ein Flockungsmittel für rote Blutkörperchen (219) in dem zweiten Fach (209) enthalten ist,

wobei, wenn die Deckelanordnung (201) an einem Behälter angebracht ist, das Flockungsmittel für rote Blutkörperchen (219) so konfiguriert ist, dass es bei Eindrücken des zweiten eindrückbaren Knopfes (213) selektiv aus dem zweiten Fach (209) in den Behälter hinein freigesetzt wird, und wobei das Flockungsmittel für rote Blutkörperchen (219) so konfiguriert ist, dass es innerhalb des Behälters ausgeflockte rote Blutkörperchen bildet.

2. Deckelanordnung nach Anspruch 1, wobei das blutgerinnende Material Aluminiumchlorid, Blutstillungspulver, Kupfersulfat, Aluminiumsulfat, Kaliumaluminium, Kaliumferrat, Eisensulfat, Ferrosulfat, Eisenchlorid oder Zinksulfat umfasst.

3. Deckelanordnung nach Anspruch 1, wobei die Deckelanordnung zur Befestigung an dem Behälter dient, bei dem es sich um einen Auffangkanister handelt.

4. Deckelanordnung nach Anspruch 1, wobei das Flockungsmittel für rote Blutkörperchen PolyDADMAC ist.

5. Deckelanordnung nach Anspruch 1, wobei die zerbrechliche Abdeckung (259, 269) eine Folie oder einen Kunststofffilm umfasst.

6. Deckelanordnung nach Anspruch 1, weiter umfassend einen Koppeladapter (208) entlang eines Umfangs der Deckelanordnung, wobei der Koppeladapter eine Gewindekonfiguration umfasst, die so konfiguriert ist, dass die Deckelanordnung an einer oberen Öffnung eines Behälters befestigt werden kann.

7. Deckelanordnung nach Anspruch 1,

wobei das erste Fach (207) eine erste Kammer umfasst und das blutgerinnende Material (250) in der ersten Kammer enthalten ist und das blutgerinnende Material (250) so konfiguriert ist, dass es eine geronnene Blutmasse bildet; und

wobei das zweite Fach (209) eine zweite Kammer umfasst, und die zweite Kammer ein Flockungsmittel für rote Blutkörperchen (219) enthält, und das Flockungsmittel für rote Blutkörperchen so konfiguriert ist, dass es ausgeflockte rote Blutkörperchen bildet.

8. Kombination der Deckelanordnung nach Anspruch 1 mit einem Auffangbehälter (30), wobei die Deckelanordnung (201) an dem Auffangbehälter befestigt ist und der Behälter Blut enthält; und wobei

die Freisetzung der Blutgerinnung (250) aus der ersten Kammer in den Behälter hinein bei Eindrücken des Knopfes die Bildung einer geronnenen Blutmasse im Behälter ermöglicht, um eine spritzerfreie Entsorgung des Blutes bereitzustellen; und

die Freisetzung des Flockungsmittels für rote Blutkörperchen (219) aus der zweiten Kammer in den Behälter hinein bei Eindrücken des Knopfes die Bildung von ausgeflockten roten Blutkörperchen im Behälter ermöglicht, wobei das Flockungsmittel für rote Blutkörperchen (219) bei Kontakt mit Blut im Behälter keine geronnene Masse bildet.

9. Kombination nach Anspruch 8, wobei die geronnene Masse eine feste Masse ist.

**10.** Kombination nach Anspruch 8,

wobei der Auffangbehälter eine Kanisterkonfiguration und ein Fassungsvermögen von 100 ml, 250 ml, 500 ml, 1.200 ml oder 5.000 ml aufweist; und
wobei die Deckelanordnung einen Koppeladapter (208) umfasst, der zur sicheren Befestigung an einer Öffnung des Auffangbehälters geeignet ist.

**11.** Kombination nach Anspruch 10, wobei der Sammelbehälter ein Volumen von etwa 1200 ml oder 5000 ml aufweist.

**12.** Kombination nach Anspruch 10, wobei der Kanister weiter eine Oberflächenbeschichtung aus einem Flockungsmittel für rote Blutkörperchen umfasst, und wobei das Flockungsmittel für rote Blutkörperchen so konfiguriert ist, dass es innerhalb des Sammelbehälters ausgeflockte rote Blutkörperchen bildet.

**13.** Kombination nach Anspruch 12, wobei das Flockungsmittel für rote Blutkörperchen PolyDADMAC umfasst.

**Revendications**

**1.** Ensemble couvercle (201) destiné à être fixé à un récipient, comprenant :

un premier compartiment (207), un premier bouton compressible (211) et un revêtement frangible (259), dans lequel le premier bouton compressible (211) et le revêtement frangible (259) définissent le premier compartiment (207), dans lequel le revêtement frangible est situé sous le premier compartiment et un matériau de coagulation sanguine (250) est contenu dans le premier compartiment (207),
dans lequel, lorsque l'ensemble couvercle (201) est fixé à un récipient, le matériau de coagulation sanguine (250) est configuré pour être libéré sélectivement du premier compartiment (207) et aller dans le récipient lors de la compression du premier bouton compressible (211), et
dans lequel le matériau de coagulation sanguine (250) est configuré pour former une masse de sang coagulé à l'intérieur du récipient ;
dans lequel l'ensemble couvercle (201) est **caractérisé en ce qu'**il comprend en outre :

un deuxième compartiment (209), un deuxième bouton compressible (213) et un revêtement frangible (269), dans lequel le revêtement frangible (269) est situé sous le deuxième compartiment (209) et un floculant de globules rouges (219) est contenu dans le deuxième compartiment (209),
dans lequel, lorsque l'ensemble couvercle (201) est fixé à un récipient, le floculant de globules rouges (219) est configuré pour être libéré sélectivement du deuxième compartiment (209) pour aller dans le récipient lors de la compression du deuxième bouton compressible (213), et dans lequel le floculant de globules rouges (219) est configuré pour former des globules rouges floculés à l'intérieur du récipient.

**2.** Ensemble couvercle selon la revendication 1, dans lequel le matériau coagulant sanguin comprend du chlorure d'aluminium, de la poudre styptique, du sulfate de cuivre, du sulfate d'aluminium, de l'alun de potassium, du ferrate de potassium, du sulfate ferrique, du sulfate ferreux, du chlorure ferrique ou du sulfate de zinc.

**3.** Ensemble couvercle selon la revendication 1, dans lequel l'ensemble couvercle est destiné à être fixé au récipient, qui est un bocal de collecte.

**4.** Ensemble couvercle selon la revendication 1, dans lequel le floculant de globules rouges est du polyDADMAC.

**5.** Ensemble couvercle selon la revendication 1, dans lequel le revêtement frangible (259, 269) comprend une feuille ou un film plastique.

**6.** Ensemble couvercle selon la revendication 1, comprenant en outre un adaptateur de couplage (208) le long d'un périmètre de l'ensemble couvercle, l'adaptateur de couplage comprenant une configuration filetée configurée pour permettre la fixation de l'ensemble couvercle à une ouverture supérieure d'un récipient.

**7.** Ensemble couvercle selon la revendication 1,

dans lequel ledit premier compartiment (207) comprend une première chambre et le matériau coagulant sanguin (250) est contenu dans la première chambre et le matériau coagulant sanguin (250) est configuré pour former une masse de sang coagulée ; et

dans lequel ledit deuxième compartiment (209) comprend une deuxième chambre, et la deuxième chambre contient un floculant de globules rouges (219), et le floculant de globules rouges est configuré pour former du sang rouge floculé.

8.  Combinaison de l'ensemble couvercle selon la revendication 1 avec un récipient de collecte (30), dans laquelle l'ensemble couvercle (201) est fixé au récipient de collecte et le récipient contient du sang ; et dans laquelle

la libération de la coagulation sanguine (250) de la première chambre pour aller dans le récipient lors de la compression du bouton permet la formation d'une masse de sang coagulée dans le récipient pour assurer l'élimination de sang sans déversement ; et

la libération du floculant de globules rouges (219) de la deuxième chambre pour aller dans le récipient lors de la compression du bouton permet la formation de globules rouges floculés dans le récipient, dans laquelle ledit floculant de globules rouges (219) ne forme pas de masse coagulée au contact du sang dans le récipient.

9.  Combinaison selon la revendication 8, dans laquelle la masse coagulée est une masse solide.

10.  Combinaison selon la revendication 8,

dans laquelle le récipient de collecte présente une configuration de bocal et une contenance de 100 ml, 250 ml, 500 ml, 1 200 ml ou 5 000 ml ; et

dans laquelle l'ensemble couvercle comprend un adaptateur de couplage (208) configuré pour être fermement fixé à une ouverture du récipient de collecte.

11.  Combinaison selon la revendication 10, dans laquelle le récipient de collecte présente un volume d'environ 1 200 ml ou 5 000 ml.

12.  Combinaison selon la revendication 10, dans laquelle le bocal comprend en outre un revêtement de surface d'un floculant de globules rouges, et dans laquelle le floculant de globules rouges est configuré pour former des globules rouges floculés à l'intérieur du récipient de collecte.

13.  Combinaison selon la revendication 12 dans laquelle le floculant de globules rouges comprend du polyDADMAC.

FIG. 1

FIG. 2

FIG. 3

FIG. 4

FIG. 5

FIG. 6

FIG. 7

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 17026106 B
- US 63055577
- DE 10343668 A1
- DE 202007003050 U1
- US 5185007 A
- WO 2021054297 A
- US 10401347 B
- US 363674
- US 16257876 B
- US 16257673 B
- US 16257876
- US 16257673
- US 15868983
- US 924547
- US 868983
- US 257876
- US 257673

**Non-patent literature cited in the description**

- **NOURI, S. et al.** Efficacy and Safety of Aluminum Chloride in Controlling External Hemorrhage: An Animal Model Study. *Iran Red Crescent Medical Journal*, 17 March 2015
- **RATERMANN, A. et al.** *Journal of Agricultural Food Chemistry*, 1980, vol. 28 (2), 438-44